# EUROPEAN PATENT SPECIFICATION

(11) **EP 0 433 629 B1**
(45) Date of publication and mention of the grant of the patent: **09.11.1994**
(21) Application number: 90121180.5
(22) Date of filing: 06.11.1990
(51) Int. Cl.: G01N 33/569

(54) **A method for the qualitative and quantitative determination of antibodies against bacterial antigens by means of the photometric measurement of agglutination**
Verfahren zur quantitativen und qualitativen Bestimmung von Antikörpern gegen bakterielle Antigene mittels photometrischer Messung der Agglutination
Procédé pour la détermination qualitative et quantitative d'anticorps contre des antigènes bactériens au moyen d'un mesurage photométrique de l'agglutination

(30) Priority: 22.12.1989 IT 2280589
(43) Date of publication of application: 26.06.1991
(73) Proprietor: DIESSE DIAGNOSTICA SENESE s.r.l., I-20123 Milano (IT)
(72) Inventor: Ricci, Antonio, I-53035 Monteriggioni, Siena (IT); Paoli, Carlo, I-50023 Impruneta, Firenze (IT)
(74) Representative: Giambrocono, Alfonso, Dr. Ing.

(56) References cited:
- EP-A- 0 161 638
- BE-A- 901 567

## Description

The present invention relates to a method for the qualitative and quantitative determination of specific antibodies (titre) by means of the measurement of the transmittance variation in mixtures containing bacterial suspensions of specific antibodies in particulate form and serum samples possibly containing said antibodies.

For example, the serodiagnosis of brucellosis and of typhoid fever passes through the detection of specific antibodies in serum that recognize the antigens in the etiological agent and react therewith, thereby causing the antigen suspension to be agglutinated.

An analytical technique that has been used up to now to detect antibodies against bacterial antigens antibodies is known as the Widal-Wright reaction. It comprises using a killed germ suspension, said germs being intact in their antigen structure (such as salmonellae for the Widal and brucellae for the Wright reaction), said suspension being incubated overnight at 37°C with scaled dilutions of the test serum in normal saline. There occurs thereby an agglutination due to the aggregation of bacterial bodies that are bounded to one another by means of the specific antibodies, which causes the sedimentation rate to be increased. The sediment can be brought again in suspension by stirring the tube, said suspension being non-homogeneous; the supernatant, before stirring, is usually clear, thereby evidencing that the agglutinated germs have been decanted. As far as the visual judgement is concerned, a test tube will be agglutination-negative when the light incident into the suspension is homogeneous. To the contrary, the agglutination can be seen as a mass of more or less coherent "granules". The titre of the sample is determined as the highest dilution thereof at which a visible agglutinate is still present.

In practice, to carry out a Widal-Wright analysis, for example, at least 5 tests (as many as the kinds of bacteria are) are carried out in test tubes with 5 or 6 different serum dilutions per test, giving a total of 25 or 30 test tubes that are to be incubated for at least eighteen hours with the killed bacteria suspension. This assay, besides being time-consuming and laborious, gives rise to a large approximation in the quantitative determination as it only relies on the discrimination capacity of the observer.

To the purpose of simplifying said analytical procedure, both slide assays and microplate assays in U-shaped wells have been developed. In the slide assays, the bacterial suspensions are stained a known technique and spotted in drop amounts. The test is carried out by observing whether within one minute agglutination occurs in the mixture of the test serum and a drop of the bacterial suspension spread over the surface of slide. Like all the slide tests, the observation is carried out under a strong illumination with rotation of the reaction mixture. Said assay is very practicable but is not able to supply a thorough measurement of the concentration of the antibodies possibly present in the test serum. The slide assay is especially advantageous for the detection of the negative tests as it provides a high sensitivity, and it is therefore used as a qualitative test for the discrimination between positive and negative samples (said test being known to the skilled persons as the "screening test").

Also the microplate assay technique is carried out with suspensions of stained germs. It relies on the classical principle of the Widal-Wright tube test. But it is miniaturized by means of the use of U-shaped wells having a capacity of about 0,3 ml. The various dilutions to be carried out are made easier by the use of multipoint pipettes and by a handier reaction vessel. The results are read by observing whether a stained button is present at the bottom of the well (no agglutination) or a homogeneous layer (agglutinated germs). The main drawback of this technique is the impossibility of observing the difference between granular agglutinate, (due, in the case of the Widal reaction, to the anti-salmonella antibodies against the lipopolysacharide antigen) and a flaky agglutinate (due to anti-salmonella antibodies against the ciliar antigen). Moreover, a low precision can be achieved in the quantitative determination of the antibody concentration. This technique too implies long incubation times.

BE 0901567 discloses a method which doses the concentration of bacterial anti-antigenic antibodies in serum samples exploiting inert particles, which may be blood cells or latex particles, whereon bacterial antigens are absorbed.

EP 161638 describes a method for dosing immunoglobulins in the test serum which uses monoclonal antibodies specifically chosen for each kind of human gamma-globulins instead of polyclonal antibodies.

There has now been surprisingly discovered a method that allows a very accurate quantitative determination of specific antibodies possibly present in serum (or plasma) samples, in very short time, to be carried out, with only one sample dilution, or two dilutions to avoid the pro-zone phenomenon in the Wright reaction.

Said method is based on the observation that when through a bacterial antigen suspension in contact with the test serum or plasma sample in a photometer cuvette a monochromatic light beam passes, the transmittance of said mixture is proportional to the number of agglutinated antigen particles which, in turn, are proportional to the concentration of specific antibodies in the test sample. Obviously, instead of transmittance the absorbance can be measured, which is inversely proportional to the antibody concentration.

In the serodiagnosis according to Widal-Wright, the bacteria suspension absorbs and/or scatters the incident monochromatic light beam, thereby causing a lowering of transmittance as compared with a clear solution, said lowering being proportional to the number of the bacteria present in the suspension. When, in suitable analytical conditions, specific antibodies-containing serum is mixed with said suspension in the same cuvette, the agglutination of the bacteria causes, as a matter of fact, a reduction of the number of particles that are in a measure of absorbing and/or scattering the light, and the transmittance will be again increased. Said data can be detected by means of instruments and, since the amount of antibodies present in the test sample modulates the agglutination amount (high titres cause many bacteria to be agglutinated, while low titres give rise to partial agglutination), from the instrumental measurement the antibody titre of the test sample can be obtained.

In fact, in the present invention a direct proportionality between the transmittance increase (or absorbance reduction) and the specific antibody titre has been observed.

The method consists in testing the samples with different kinds of bacteria with no previous dilution whilst in the known art 25 to 30 dilutions are necessary) in cuvettes that are put in the path of a light beam having a wave-length of from 260 to 1100 nm, preferably from 400 to 800 nm, most preferably of 565 nm. To the purpose of increasing the method sensitivity, the bacteria can be previously stained by using a dye the absorbance peak of which coincides with wave-length used for the photometric readings. The assay, after mixing the bacterial suspension with the serum possibly containing specific antibodies, can be carried out by incubating the mixture for 30 seconds to 10 minutes, preferably 5 minutes. The time for the analysis is thus drastically reduced.

The intensity of the light beam leaving the cuvette is first measured. Said light intensity can be determined with the detector on the same axis as the incident beam (angle of 180°) or at different angles (for example at an angle of 90° with nephelometric reading). After the reaction, the higher the amount of emerging light (transmittance), the higher is the number of specific antibodies present in the test serum sample that have reacted with the bacteria in the suspension. If the photometric readings are carried out in absorbance, after the reaction the absorbance will be lowered relative to the starting one. The increase in transmittance or reduction in absorbance can be monitored not only at the end point but also in a kinetic mode, by comparing it with the transmittance value at the beginning of the reaction. If the assay is negative, the final transmittance or absorbance will be unchanged relative to the starting value.

If the instrument is calibrated by measuring the transmittance variations of reaction mixtures in which samples with known antibody titre have been used, an immediate quantitative reading can be achieved. It is therefore clear that in the practice of the invention it is possible to assay one single sample dilution per antigen suspension, thereby determining effectively and quickly the antibody titre of the test sample.

An example of an embodiment of the invention follows. Said example is illustrative and should not be construed as limiting the invention.

### EXAMPLE

### Quantitative determination of anti-brucella antibodies

The reagent consist of a Brucella abortus suspension stained so as to maximize the absorbance at the desired wave- length, said wave-length coinciding with the absorbance peak of the employed dye. The concentration of the suspension is optimized so as to have a final transmittance of the order of 25% of full scale (0-100%). In micro-cuvettes for photometry equipped with stirrer, 800 µL of antigen suspension are mixed with 20 µL of test serum. Under continuous stirring, the transmittance is read at zero reaction time and after 5 minutes. The difference of the two transmittance values read is then made.

For titres lower then or equal to 1/80 checked by means of the reference Wright test in test tube, the transmittance difference is almost nil as the system is calibrated for the detection of titres higher then 1/80; it should be borne in mind that only titres equal to or higher then 1/160 are to be considered positive and safe for the disease diagnosis.

Using 4 calibration serums with known titres between 1/100 and 1/1600, a calibration curve is plotted with the transmittance variation on the y-axis as they are detected by the instrument, against the related titre on the x-axis.

The test sample with known titre is processed as a calibration serum. At the end of the measurement, the transmittance difference measured at the beginning of the reaction is plotted in the graph that has been obtained with the known titre calibration serums. By interpolation the titre of the test sample can be obtained.

With a suitable, computer-controlled instrument assembly the values of the calibration samples can be stored and the calculations concerning the test samples can be carried out automatically. The hand-work for the construction of calibration graphs and the interpolation of the unknown values can be avoided.

## Claims

1. A process for the qualitative and quantitative determination of antibodies in serum, characterized in that the measurement of the transmittance variation of mixtures consisting of a specific bacterial antigen suspension which comprises killed or inactived germs treated with a dye having an absorbance peak at the wavelength chosen for the photometric reading and the test serum is carried out by photometrically reading said mixtures at a wave-length of between 260 and 1100 nm, the measured transmittance variation being proportional to the antibody concentration in the test serum.

2. A process as claimed in claim 1, characterized in that the photometric readings are carried out at a wave-length of between 400 and 800 nm

3. A process as claimed in claim 2, characterized in that the photometric readings are carried out at a wave-length of 565 nm.

4. A process as claimed in claims 1 to 3, in which the measurement of the transmittance variation is carried out by using a detector lying at an angle different from 180° relative to the incident beam.

5. A process as claimed in claims 1 to 3, in which the measurement of the absorbance variation is carried out by using a detector lying at an angle different from 180° relative to the incident beam.

6. A process as claimed in claims 1 to 5, characterized in that said serum is diluted with normal saline.

## Patentansprüche

1. Verfahren zur qualitativen und quantitativen Bestimmung von Antikörpern im Serum, dadurch **gekennzeichnet**, daß die Messung der Transmissionsvariation von Gemischen, bestehend aus einer spezifischen bakteriellen Antigensuspension, die abgetötete oder inaktivierte Keime, die mit einem Farbstoff, der einen Absorptionspeak bei der Wellenlänge, die für die photometrische Ablesung verwendet wurde, besitzt, behandelt wurden, umfaßt, und dem Testserum, durch photometrische Ablesung der Gemische bei einer Wellenlänge zwischen 260 und 1100 nm durchgeführt wird, wobei die gemessene Transmissionsvariation der Antikörperkonzentration in dem Testserum proporptional ist.

2. Verfahren nach Anspruch 1, dadurch **gekennzeichnet**, daß die photometrischen Ablesungen bei einer Wellenlänge zwischen 400 und 800 nm durchgeführt werden.

3. Verfahren nach Anspruch 2, dadurch **gekennzeichnet**, daß die photometrischen Ablesungen bei einer Wellenlänge von 565 nm durchgeführt werden.

4. Verfahren nach den Ansprüchen 1 bis 3, dadurch **gekennzeichnet**, daß die Messung der Transmissionsvariation unter Verwendung eines Detektors durchgeführt wird, der in einem Winkel, der sich von 180°, bezogen auf den einfallenden Strahl, unterscheidet, angeordnet ist.

5. Verfahren nach den Ansprüchen 1 bis 3, dadurch **gekennzeichnet**, daß die Messung der Absorptionsvariation unter Verwendung eines Detektors, der in einem Winkel, der sich von 180°, bezogen auf den einfallenden Strahl, unterscheidet, angeordnet ist, durchgeführt wird.

6. Verfahren nach den Ansprüchen 1 bis 5, dadurch **gekennzeichnet**, daß das Serum mit normaler Kochsalzlösung verdünnt wird.

## Revendications

1. Procédé pour doser qualitativement et quantitativement les anticorps dans le sérum, caractérisé en ce que la mesure de la variation du facteur de transmission de mélanges constitués d'une suspension spécifique d'antigènes bactériens, qui comprend des germes tués ou inactivés, traités par un colorant ayant un pic d'absorption à la longueur d'onde choisie pour la lecture photométrique, et d'un sérum d'essai, est mise en oeuvre par photométrie des mélanges à une longueur d'onde de 260 à 1.100 nm, la variation du facteur de transmission telle que mesurée étant proportionnelle à la concentration de l'anticorps dans le sérum d'essai.

2. Procédé selon la revendication 1, caractérisé en ce que les lectures photométriques sont effectuées à une longueur d'onde de 400 à 800 nm.

3. Procédé selon la revendication 2, caractérisé en ce que les lectures photométriques sont effectuées à une longueur d'onde de 565 nm.

4. procédé selon les revendications 1 à 3, dans lequel la mesure de la variation du facteur de transmission est effectuée par utilisation d'un détecteur faisant par rapport au faisceau incident un angle différent de 180°.

5. Procédé selon les revendications 1 à 3, dans lequel la mesure de la variation du facteur d'absorption est effectuée par utilisation d'un détecteur faisant par rapport au faisceau incident un angle différent de 180°.

6. Procédé selon les revendications 1 à 5, caractérisé en ce que le sérum est dilué avec une solution salée normale.
